# FASCICULE DE BREVET EUROPEEN

(11) **EP 1 336 422 B1**
(45) Date de publication et mention de la délivrance du brevet: **09.09.2015**
(21) Numéro de dépôt: 03290270.2
(22) Date de dépôt: 04.02.2003
(51) Int. Cl.: A61B 5/00, A61B 5/053

(54) **Dispositif médical actif, notamment stimulateur cardiaque, comprenant des moyens perfectionnés de détection et de traitement des troubles ventilatoires du sommeil**
Aktive medizinische Vorrichtung, insbesondere Herzschrittmacher, mit verbesserten Mitteln zur Erkennung und Behandlung von Beatmung-Schlafstörungen
Active medical device, in particular pacemaker, having improved means for detecting and treating ventilation sleep disorders

(30) Priorité: 15.02.2002 FR 0201936
(43) Date de publication de la demande: 20.08.2003
(73) Titulaire: ELA MEDICAL, 92541 Montrouge (FR)
(72) Inventeur: Poezevera, Yann, 91080 Courcouronne (FR)
(74) Mandataire: Dupuis-Latour, Dominique

(56) Documents cités:
- EP-A- 0 493 222
- EP-A- 0 702 977
- EP-A- 1 151 718
- EP-A2- 0 940 155
- US-A- 5 919 209
- US-A- 6 015 388
- US-A- 6 141 590

## Description

L'invention concerne les "dispositifs médicaux implantables actifs" tels que définis par la directive 90/385/CEE du 20 juin 1990 du Conseil des communautés européennes, notamment les stimulateurs cardiaques, défibrillateurs et/ou cardioverteurs permettant de délivrer au coeur des impulsions électriques de faible énergie pour le traitement des troubles du rythme cardiaque.

L'invention concerne plus particulièrement le suivi de l'activité respiratoire et le contrôle corrélatif de la stimulation cardiaque.

On connaît, notamment d'après le EP-A-0 493 222 (Ela Médical), des stimulateurs cardiaques comprenant des moyens de mesure de l'activité respiratoire du patient, plus précisément de la ventilation-minute (c'est-à-dire du produit de la fréquence respiratoire par l'amplitude ventilatoire). Ce dernier paramètre y est utilisé pour asservir la fréquence d'application des impulsions de stimulation, non pour réaliser une analyse de la ventilation permettant de détecter certains troubles particuliers survenant pendant le sommeil.

Pour cela, on a déjà proposé des dispositifs permettant de diagnostiquer divers troubles respiratoires tels que les apnées révélatrices d'une pathologie connue sous le nom de "syndrome d'apnée du sommeil (SAS)" - une telle apnée étant définie comme étant une pause respiratoire de durée supérieure à dix secondes et survenant pendant une phase de sommeil du patient. Un SAS peut être également défini par une récurrence importante d'hypopnées.

On sait également détecter des troubles révélés par une variation périodique et anormale du profil respiratoire, où des phases d'hyperventilation alternent avec soit des phases de respiration normale ou d'hypoventilation (trouble connu sous l'appellation de "respiration périodique" ou PB, *Periodic Breathing),* soit des pauses respiratoires (trouble connu sous l'appellation de "dyspnée de Cheyne-Stokes" ou "respiration de Cheyne-Stokes", ou encore CSR, *Cheyne-Stokes Respiration*). Les différentes phases alternées ont chacune une durée de quelques cycles à quelques dizaines de cycles respiratoires, c'est-à-dire de quelques secondes ou dizaines de secondes, pouvant même dépasser une minute. Un trouble voisin est l'hypopnée, qui est une décroissance importante de la ventilation-minute, par exemple plus de 50 %, par rapport à une moyenne de référence antérieure (l'hypopnée se distingue de l'apnée ou pause respiratoire, qui est un arrêt temporaire de la fonction respiratoire).

Le EP-A-1 151 718 (Pacesetter) décrit un appareil permettant d'analyser ainsi le rythme respiratoire et de diagnostiquer de tels troubles du sommeil.

Le point de départ de l'invention réside dans la constatation que les hypopnées, qui peuvent survenir de façon sporadique indépendamment de troubles cycliques tels que PB ou CSR évoqués plus haut, peuvent en particulier induire une désaturation en oxygène du sang susceptible de provoquer le réveil du patient mais aussi, et surtout, une tachycardie sinusale transitoire destinée à compenser cette désaturation.

L'un des buts de l'invention est de proposer un dispositif qui puisse détecter ce type de trouble pendant le sommeil du patient et assurer une thérapie appropriée en agissant sur la fréquence de délivrance d'impulsions de stimulation cardiaque lorsqu'un tel trouble est détecté et que certains critères spécifiques sont remplis, ceci de manière à anticiper et éviter les conséquences du trouble, notamment éviter le réveil du patient et les anomalies du rythme cardiaque entraînées par la désaturation en oxygène.

Le EP 0 940 155 A décrit un tel dispositif comprenant des moyens de détection d'apnée et des moyens pour produire une stimulation forcée à un rythme supérieur en cas d'apnée détectée. Cette stimulation provoque un réveil du patient qui met fin à l'épisode d'apnée. Le dispositif comprend par ailleurs un capteur d'activité permettant d'activer mécanisme seulement pendant les phases de repos.

Le problème de l'invention est de remédier au cas où l'accélération de la fréquence cardiaque par la stimulation déclenchée soit trop importante et provoque alors un réveil systématique du patient.

L'invention propose à cet effet un dispositif du type général divulgué par le EP 0 940 155 A précité, correspondant au préambule de la revendication 1, et comportant les éléments distinctifs énoncés dans la partie caractérisante de cette revendication 1.

Les sous-revendications visent des formes de mise en oeuvre particulières avantageuses.

On va maintenant décrire plus en détail l'invention, dans le cadre d'un exemple de mise en oeuvre.

Comme on l'a indiqué plus haut, la pause respiratoire n'est pas le seul trouble du sommeil qui induise une désaturation en oxygène du sang ; la réduction importante (sans pause) du volume respiratoire, ou hypopnée, en est un autre.

Pour la mise en oeuvre de l'invention, la survenue de telles hypopnées est détectée par analyse de la variation du signal de ventilation-minute.

Ce signal de ventilation-minute est, de manière bien connue, mesuré à partir des paramètres d'amplitude et de périodicité des cycles respiratoires successifs. L'analyse cycle à cycle est opérée suivant une méthode standard, par exemple décrite dans le EP-A-0 493 222 précité, auquel on pourra se référer pour de plus amples détails sur la manière dont le signal représentatif de l'activité respiratoire est recueilli et analysé, notamment pour en dériver l'information de ventilation-minute (signal dit VE ou MV),

Le signal MV est un paramètre à prépondérance physiologique obtenu par une mesure intrathoracique d'impédance. Cette mesure est opérée entre deux électrodes disposées sur, ou dans, la cage thoracique, ou entre une électrode (par exemple une électrode de stimulation, si le dispositif implanté est un stimulateur cardiaque) et un boîtier du dispositif. L'impédance est mesurée par injection d'un courant constant de quelques centaines de microampères, à une fréquence de quelques Hertz, typiquement 8 Hz. Cette technique est par exemple décrite par Bonnet JL et coll., Measurement of Minute-Ventilation with Different DDDR Pacemaker Electrode Configurations, PACE, Vol. 21, 98, Part 1, et elle est mise en oeuvre Configurations, *PACE*, Vol. 21, 98, Part 1, et elle est mise en oeuvre dans les appareils *Chorus RM 7034* d'ELA Médical.

On notera toutefois que l'invention n'est pas limitée aux seuls dispositifs procédant par analyse d'un signal physiologique de ventilation-minute, bien qu'il s'agisse là de la configuration la plus courante et que, pour cette raison, la présente description sera faite dans le cadre de cet exemple. Ainsi, l'invention s'applique aussi bien à des dispositifs mettant en oeuvre d'autres types de capteurs physiologiques tels que capteurs de pH, capteurs de mesure de la saturation en oxygène du sang, etc.

Pour détecter une hypopnée, le dispositif compare entre elles des moyennes glissantes de la ventilation-minute, moyennes établies par exemple sur une durée de 10 secondes. Si, entre deux moyennes consécutives, une décroissance importante de la ventilation-minute est détectée, par exemple une décroissance de plus de 50 %, alors le dispositif comptabilise une hypopnée.

En variante, le dispositif peut établir la moyenne de la ventilation-minute sur, par exemple, quatre cycles consécutifs et comparer cette valeur à la moyenne des, par exemple, quatre cycles précédents (c'est-à-dire que la moyenne est établie sur un nombre donné de cycles respiratoires, au lieu de l'être sur un laps de temps prédéterminé).

On notera que les intervalles de temps, ou les nombres de cycles, sur lesquels sont calculées les moyennes peuvent être les mêmes pour les deux valeurs à comparer - comme dans l'exemple ci-dessus -, ou bien être différents : on peut ainsi par exemple comparer la moyenne des deux dernières valeurs du signal MV à celle des quatre valeurs précédentes, ou encore la dernière valeur du signal MV à la moyenne des quatre valeurs précédentes (dans ce dernier cas, la "moyenne" est obtenue à partir d'une valeur unique, c'est-à-dire que la notion de "moyenne de N valeurs" est entendue au sens large avec N ≥ 1).

Le dispositif détermine également la durée de l'hypopnée détectée, durée qui sera égale au nombre de périodes glissantes consécutives (ou au nombre de cycles consécutifs, dans la variante exposée ci-dessus) pour lesquelles la valeur de la ventilation-minute demeurera inférieure au seuil de comparaison de la dernière moyenne non hypopnéique.

En cas de survenue d'une ou plusieurs hypopnées, lorsque certains critères sont vérifiés le dispositif modifie un paramètre de fonctionnement, typiquement la fréquence de délivrance des impulsions de stimulation dans le cas d'un stimulateur cardiaque.

Les critères conditionnant cette modification du paramètre de fonctionnement peuvent être notamment :
- la durée cumulée des hypopnées comptabilisées sur une période donnée (par exemple sur une heure) est supérieure à un seuil (par exemple supérieure à 10 minutes) ; ou
- le nombre des hypopnées comptabilisées sur une période donnée (par exemple sur une heure) est supérieur à un seuil donné (par exemple supérieur à 20) ; ou
- la durée d'une seule hypopnée est supérieure à un seuil prédéterminé (par exemple supérieure à 2 minutes).

Dans l'un ou l'autre de ces cas, une désaturation en oxygène est suspectée, et la fréquence de stimulation est accélérée.

Le but de l'accélération de la fréquence cardiaque est d'augmenter le débit cardiaque, tout en évitant le réveil du patient et une possible tachycardie sinusale - qui sont les deux conséquences principales de la désaturation en oxygène induite par le trouble ventilatoire.

Toutefois, il est possible que l'accélération de la fréquence cardiaque par la stimulation ainsi déclenchée soit trop importante, et provoque alors un réveil systématique du patient.

Pour éviter qu'une telle situation ne se répète, et fasse alors perdre tout le bénéfice du traitement, le dispositif analyse les réveils éventuels consécutifs à une augmentation de la fréquence de stimulation.

On connaît diverses techniques pour opérer la discrimination entre éveil et sommeil. Le EP-A-0 719 568 (Ela Médical) opère cette discrimination par analyse du signal de ventilation-minute, en calculant une moyenne du signal MV sur un nombre donné de cycles respiratoires, par exemple les 128 derniers cycles, en comparant cette valeur moyenne avec une valeur de référence, par exemple la moyenne du signal MV sur les dernières 24 heures. On peut également utiliser un capteur à réponse rapide, typiquement un capteur d'activité ou d'accélération ("capteur G"), dont le signal permet de détecter des mouvements du patient ; l'information de ce type de capteur n'est en elle-même pas très spécifique des phases d'éveil et de sommeil, mais on sait combiner les signaux délivrés par un capteur G et un capteur MV pour en déduire des informations signifiantes, comme cela est décrit par exemple dans les EP-A-0 750 920 (Ela Médical) et EP-A-0 770 407 (Ela Médical), auxquels on pourra se référer pour de plus amples détails.

La détection du réveil est utilisée de la manière suivante dans le cadre de l'invention.

Si un réveil est détecté dans un délai court (par exemple 5 minutes) après le début de l'accélération de la fréquence cardiaque par stimulation, alors un compteur de réveils est incrémenté.

Lors de la phase de sommeil suivante, si, de nouveau, la fréquence cardiaque est augmentée par stimulation suite à une hypopnée et que cette accélération induit un réveil dans les mêmes conditions que précédemment, le compteur est à nouveau incrémenté.

Si la valeur du compteur de réveils atteint une valeur prédéterminée, par exemple cinq réveils, alors la fréquence-cible de stimulation cardiaque en réponse à une détection d'hypopnée est réduite d'un pas, par exemple réduite de 5 cpm. Le processus est répété et cette réduction est opérée de façon itérative jusqu'à ce que l'augmentation de la fréquence cardiaque devienne égale à 5 cpm (par exemple) au-dessus du rythme spontané du patient, car au-delà la thérapeutique pourrait être considérée comme inefficace.

Avantageusement, le dispositif prévoit également de détecter certains cas de faux positifs.

En effet, les capteurs de détection de la désaturation en oxygène, et notamment de la ventilation-minute, peuvent présenter des faux positifs, par exemple pour certaines positions du patient lors de son sommeil, du fait d'une modification de la respiration, qui devient essentiellement abdominale et qui peut induire une réduction importante des variations du volume respiratoire thoracique.

Dans ce cas, le capteur MV enregistrerait - à tort - des phases de pauses respiratoires, ou d'hypopnées, présentant des durées anormalement longues pouvant atteindre plusieurs minutes.

On prévoit avantageusement que, dans ce cas, ces épisodes particuliers soient exclus du comptage des événements susceptibles de déclencher une modification des paramètres de fonctionnement du dispositif, dès lors que leur durée dépasse un seuil, par exemple un seuil d'une minute pour les pauses respiratoires, et un seuil de cinq minutes pour les hypopnées.

## Revendications

1. Un dispositif médical implantable actif, comprenant :
- des moyens de délivrance d'impulsions de stimulation cardiaque ;
- des moyens d'analyse d'activité respiratoire aptes à délivrer un signal d'activité ventilatoire du patient et à détecter la survenue d'hypopnées à partir de ce signal ;
- des moyens de commande aptes à augmenter la fréquence des impulsions de stimulation à une fréquence-cible en cas de détection d'hypopnées ; et
- des moyens de détection des phases d'éveil et de sommeil du patient, **caractérisé en ce que** :
- les moyens de détection des phases d'éveil et de sommeil sont aptes à incrémenter un compteur de réveils si après le début de ladite augmentation de la fréquence des impulsions de stimulation un réveil est détecté après un délai court; et
- les moyens de commande sont aptes à réduire la fréquence-cible si la valeur du compteur de réveils atteint une valeur prédéterminée.

2. Le dispositif de la revendication 1, dans lequel les moyens de commande sont aptes à réduire de façon itérative la fréquence-cible par pas successifs.

3. Le dispositif de la revendication 2, dans lequel les moyens de commande sont aptes à réduire de façon itérative la fréquence-cible jusqu'à ce que cette fréquence-cible devienne égale à une valeur donnée au-dessus du rythme spontané du patient.

4. Le dispositif de la revendication 1, dans lequel les moyens d'analyse comprennent :
- des moyens pour calculer à intervalles réguliers des moyennes glissantes du signal d'activité ventilatoire,
- des moyens pour comparer entre elles les valeurs des moyennes glissantes successives ainsi calculées, et
- des moyens pour détecter la survenue d'une hypopnée lorsque, pour deux moyennes glissantes successives, l'écart entre ces moyennes franchit un seuil de comparaison prédéterminé.

5. Le dispositif de la revendication 4, dans lequel les moyennes glissantes sont calculées chacune sur un laps de temps prédéterminé.

6. Le dispositif de la revendication 4, dans lequel les moyennes glissantes sont calculées chacune sur un nombre N, avec N ≥ 1, de cycles respiratoires prédéterminé:

7. Le dispositif de la revendication 1, dans lequel les moyens d'analyse sont des moyens également aptes, après survenue d'une hypopnée; à déterminer la durée de l'hypopnée ainsi détectée.

8. Le dispositif des revendications 4 et 7 prises en combinaison, dans lequel les moyens d'analyse déterminent la durée de l'hypopnée par comptage du nombre de cycles respiratoires consécutifs pour lesquels la moyenne du signal d'activité ventilatoire demeure inférieure audit seuil prédéterminé de comparaison de la dernière moyenne non hypopnéique.

9. Le dispositif des revendications 4 et 7 prises en combinaison, dans lequel les moyens d'analyse déterminent la durée de l'hypopnée par comptage du nombre de périodes de calcul des moyennes glissantes pour lesquelles la moyenne du signal d'activité ventilatoire demeure inférieure audit seuil prédéterminé de comparaison de la dernière moyenne non hypopnéique.

10. Le dispositif des revendications 4 et 7 prises en combinaison, dans lequel les moyens de commande sont des moyens aptes à augmenter la fréquence de stimulation lorsque la durée cumulée des hypopnées comptabilisées sur une période donnée est supérieure à un seuil prédéterminé.

11. Le dispositif des revendications 4 et 7 prises en combinaison, dans lequel les moyens de commande sont des moyens aptes à augmenter la fréquence de stimulation lorsque le nombre d'hypopnées comptabilisées sur une période donnée est supérieur à un seuil prédéterminé.

12. Le dispositif de la revendication 7, dans lequel les moyens d'analyse sont également aptes à discriminer phases de pauses respiratoires et phases d'hypopnées, et à suspendre la détermination de la durée de l'hypopnée pendant les phases de pauses respiratoires.

## Patentansprüche

1. Implantierbare, aktive medizinische Vorrichtung, umfassend:
- Mittel zur Abgabe von Impulsen zur Herzstimulation;
- Mittel zur Analyse der Atmungsaktivität, die geeignet sind, ein Signal der Beatmungsaktivität des Patienten abzugeben und das Auftreten von Hypopnoen ausgehend von diesem Signal zu erfassen;
- Mittel zur Steuerung, die geeignet sind, beim Erfassen von Hypopnoen die Frequenz der Stimulationsimpulse auf eine Zielfrequenz zu erhöhen; und
- Mittel zur Erfassung der Wach- und Schlafphasen des Patienten, **dadurch gekennzeichnet, dass**:
- die Mittel zur Erfassung der Wach- und Schlafphasen geeignet sind, einen Aufwachzähler nach Beginn der Erhöhung der Frequenz der Impulse zu inkrementieren, wenn ein Aufwachen nach einer kurzen Stimulationsverzögerung erfasst wird; und
- die Mittel zur Steuerung geeignet sind, die Zielfrequenz zu reduzieren, wenn der Wert des Aufwachzählers einen vorbestimmten Wert erreicht.

2. Vorrichtung nach Anspruch 1, wobei die Mittel zur Steuerung geeignet sind, die Zielfrequenz wiederholt schrittweise zu reduzieren.

3. Vorrichtung nach Anspruch 2, wobei die Mittel zur Steuerung geeignet sind, die Zielfrequenz wiederholt zu reduzieren, bis diese Zielfrequenz gleich einem gegebenen Wert oberhalb des Eigenrhythmus des Patienten wird.

4. Vorrichtung nach Anspruch 1, wobei die Mittel zur Analyse umfassen:
- Mittel zur Berechnung in regelmäßigen Abständen der gleitenden Mittelwerte des Signals der Beatmungsaktivität,
- Mittel zum Vergleich der Werte der auf diese Weise berechneten, aufeinander folgenden gleitenden Mittelwerte untereinander, und
- Mittel zur Erfassung des Auftretens einer Hypopnoe, wenn für zwei aufeinander folgende gleitende Mittelwerte der Abstand zwischen diesen Mittelwerten einen vorbestimmten Vergleichsschwellenwert übersteigt.

5. Vorrichtung nach Anspruch 4, wobei die gleitenden Mittelwerte jeweils über einen vorbestimmten Zeitabschnitt berechnet werden.

6. Vorrichtung nach Anspruch 4, wobei die gleitenden Mittelwerte jeweils über eine Anzahl N, mit N ≥ 1, von vorbestimmten Atemzügen berechnet werden.

7. Vorrichtung nach Anspruch 1, wobei die Mittel zur Analyse Mittel sind, die auch geeignet sind, nach dem Auftreten einer Hypopnoe, die Dauer der auf diese Weise erfassten Hypopnoe zu bestimmen.

8. Vorrichtung der Ansprüche 4 und 7 in Kombination, wobei die Mittel zur Analyse die Dauer der Hypopnoe durch Zählen der Anzahl der aufeinander folgenden Atemzüge für die der Mittelwert des Signals der Beatmungsaktivität unterhalb des vorbestimmten Vergleichsschwellenwerts des letzten Nicht-Hypnopoen-Mittelwerts bleibt, bestimmen.

9. Vorrichtung der Ansprüche 4 und 7 in Kombination, wobei die Mittel zur Analyse die Dauer der Hypopnoe durch Zählen der Anzahl der Berechnungszeiträume der gleitenden Mittelwerte für die der Mittelwert des Signals der Beatmungsaktivität unterhalb des vorbestimmten Vergleichsschwellenwerts des letzten Nicht-Hypnopoen-Mittelwerts bleibt, bestimmen.

10. Vorrichtung nach den Ansprüchen 4 und 7 in Kombination, wobei die Mittel zur Steuerung Mittel sind, die geeignet sind, die Stimulationsfrequenz zu erhöhen, wenn die kumulierte Dauer der über einen gegebenen Zeitraum gezählten Hypopnoen größer ist als ein vorbestimmter Schwellenwert.

11. Vorrichtung nach den Ansprüchen 4 und 7 in Kombination, wobei die Mittel zur Steuerung Mittel sind, die geeignet sind, die Stimulationsfrequenz zu erhöhen, wenn die Anzahl der über einen gegebenen Zeitraum gezählten Hypopnoen größer ist als ein vorbestimmter Schwellenwert.

12. Vorrichtung nach Anspruch 7, wobei die Mittel zur Analyse auch geeignet sind, Phasen von Atmungspausen und Hypopnoephasen zu unterscheiden, und die Bestimmung der Dauer der Hypopnoe während der Atmungspausenphasen zu unterbrechen.

## Claims

1. An active implantable medical device, comprising:
- cardiac stimulation pulse delivery means;
- respiratory activity analysis means adapted to deliver a signal of the patient ventilator activity and to detect the occurrence of hypopneas from this signal;
- control means adapted to increase the frequency of the stimulation pulses to a target frequency in case of detection of hypopneas; and
- patient waking and sleep phase detection means, **characterized in that**:
- the waking and sleep phase detection means are adapted to increment a wake-up counter if, after the beginning of said increase of the stimulation pulse frequency, a wake-up is detected after a short delay, and
- the control means are adapted to reduce the target frequency if the value of the wake-up counter reaches a predetermined value.

2. The device of claim 1, wherein the control means are adapted to reduce iteratively the target frequency by successive steps.

3. The device of claim 2, wherein the control means are adapted to reduce iteratively the target frequency until this target frequency becomes equal to a given value above the spontaneous rhythm of the patient.

4. The device of claim 1, wherein the analysis means comprise:
- means for calculating, at regular intervals, sliding averages of the ventilator activity signal,
- means for comparing between each other the values of the so-calculated successive sliding averages, and
- means for detecting the occurrence of a hypopnea when, for two successive sliding averages, the difference between these averages crosses a predetermined threshold of comparison.

5. The device of claim 4, wherein the sliding averages are each calculated over a predetermined lapse of time.

6. The device of claim 4, wherein the sliding averages are each calculated over a predetermined number N, with N ≥ 1, of respiratory cycles.

7. The device of claim 1, wherein the analysis means are means also adapted, after the occurrence of a hypopnea, to determine the duration of the so-detected hypopnea.

8. The device of claims 4 and 7 taken in combination, wherein the analysis means determine the duration of the hypopnea by counting the number of consecutive respiratory cycles for which the average of the ventilator activity signal remains lower than said predetermined threshold of comparison of the last non-hypopneic average.

9. The device of claims 4 and 7 taken in combination, wherein the analysis means determine the duration of the hypopnea by counting the number of periods of calculation of the sliding averages for which the average of the ventilator activity signal remains lower than said predetermined threshold of comparison of the last non-hypopneic average.

10. The device of claims 4 and 7 taken in combination, wherein the control means are means adapted to increase the stimulation frequency when the cumulated duration of the hypopneas counted over a given period is higher than a predetermined threshold.

11. The device of claims 4 and 7 taken in combination, wherein the control means are means adapted to increase the stimulation frequency when the number of hypopneas counted over a given period is higher than a predetermined threshold.

12. The device of claim 7, wherein the analysis means are also adapted to discriminate respiratory pause phases and hypopnea phases, and to suspend the determination of the hypopnea duration during the respiratory pause phases.
